# EUROPEAN PATENT SPECIFICATION

(11) **EP 3 376 994 B1**
(45) Date of publication and mention of the grant of the patent: **12.08.2026**
(21) Application number: 16866729.3
(22) Date of filing: 21.11.2016
(51) Int. Cl.: A61D 7/00, A01K 13/00, G01F 11/02, A61M 5/19, A61M 5/315

(54) **AN APPLICATOR**
APPLIKATOR
APPLICATEUR

(30) Priority: 19.11.2015 NZ 15714332
(43) Date of publication of application: 26.09.2018
(73) Proprietor: Datamars SA, 6814 Lamone (CH)
(72) Inventor: UDY, Brendan Lance, Te Kowhai 3288 (NZ); CALDER, David Bain, Ohaupo 3881 (NZ)
(74) Representative: M. Zardi & Co S.A.
(86) International application number: PCT/NZ2016/050185
(87) International publication number: WO 2017/086807

(56) References cited:
- WO-A1-2010/043317
- FR-A1- 3 027 793
- GB-A- 905 389
- NZ-A- 521 084
- US-A- 3 016 897
- US-A- 3 831 602
- US-A- 4 245 757
- US-A- 4 673 395
- US-A- 5 865 811
- US-A1- 2015 119 810
- US-A1- 2015 128 873
- US-B1- 6 475 193

## Description

The present invention relates to applicators for fluids.

### Background

US 4,673,395 and US 3,016,897 each disclose a doser with two barrels for dosing from two volumes at the same time.

Animal remedies for sheep, cattle, swine and the like are applied by a number of methods including topical or "pour-on" application, oral application, injection and nasal infusion. Each of these is typically dispensed from a "pistol grip" style applicator.

Typically such applicators have a piston or plunger which can be reciprocated within a barrel by squeezing and releasing a first handle relative to a second handle. The liquid to be dispensed is drawn into the barrel through an inlet via a one way inlet valve when the plunger is withdrawn inside the barrel, and is dispensed through a nozzle (or a needle) via an outlet valve when the plunger is extended towards the outlet valve. Such an applicator is described in the applicant's New Zealand patent No. 521084.

In some cases the treatment given to the animal comprises two different liquids. The liquids may be mixed together within the applicator, or may be applied separately but at the same time. It may be desirable to control the dose of each liquid independently of the dose of the other.

Some applicators of the prior art comprise two parallel fluid flow systems which are operated by a single handle or trigger. In order to "prime" the applicator, the handle or trigger is operated repeatedly until each of the parallel systems is filled with a respective liquid. However, if one of the two systems fills sooner than the other (for example if the length of the conduits feeding the barrels are of different lengths, or if the parallel systems are set to express different doses), then wastage of at least one of the liquids can occur, as the priming action must be repeated until the last system to fill is ready for use. In many cases the liquids in question may be expensive, and so this waste may incur a significant expense for the user.

Many applicators of the prior art are provided with mechanisms for controlling the dose expressed by the applicator. NZ521084 describes a mechanism comprising a cylindrical dosage control part which is provided with a plurality of stopping ribs, each of a different length. Rotation of the dosage control part allows selection of which of the stopping ribs is engaged by a rib provided on the plunger, and therefor allows adjustment of the maximum stroke of the plunger.

Notably, the dosage control part encircles the plunger, and so the plunger must be of adequate length to allow the plunger to achieve its full stroke (dependent on the setting of the dosage control part) before the mechanism which actuates the plunger comes into contact with the dosage control part.

Often a user will need to treat a large number of animals. Accordingly, it is important that the applicator be as compact as possible so as to be easy to use and to avoid user fatigue. Applicators may also require periodic servicing of various inlet and outlet valves in order to stay in good working condition. It is desirable for this servicing to be achievable as quickly and simply as possible. However, many applicators of the prior art have inlet and outlet valves located at a plurality of locations, requiring significant disassembly of the applicator for the valves to be serviced.

US 2015/128873, US 2015/119810 and WO 2010/043317 are other examples of applicators. The reference to any prior art in the specification is not, and should not be taken as, an acknowledgement or any form of suggestion that the prior art forms part of the common general knowledge in any country.

### Object of the Invention

It is an object of a preferred embodiment of the invention to provide an applicator which will overcome or ameliorate at least one problem with such applicators at present, or which will at least provide a useful choice.

### Brief Summary of the Invention

An applicator according to the present invention is claimed in claim 1.

Advantageous embodiments of the applicator according to the present invention are claimed in claims 2 to 9.

A non-therapeutic method of operating an applicator according to the present invention is claimed in claim 10.

The **applicator** as disclosed comprises:
- at least one barrel;
- a piston moveable within the at least one barrel between a variable first position and a second position, wherein, in use, movement of the piston towards the first position can draw a first fluid into the barrel, and movement of the piston towards the second position can force the first fluid out of the barrel;
- at least one fluid inlet valve to allow the fluid to flow into the at least one barrel at least under action of the piston, and at least one fluid outlet valve to allow the first fluid to flow out of the at least one barrel at least under action of the piston;
- biasing means to bias the piston toward the first position; characterised in that
- a dosage control part that slides linearly on a longitudinal axis of the at least one barrel, and can lock in any one of a plurality of notches that each correspond to a dosage mark to abut an abutment portion which in turn defines the variable first position of the piston and therefore a dosage of the first fluid; and
- piston actuating means operable to move the piston towards the second position to dispense the first fluid from the applicator,
Wherein a user may set the dosage of the applicator via the dosage control part.

Preferably the abutment portion is on, or connected to, the piston.

Preferably the piston comprises a piston head and a piston rod connected to or integral with the piston head, wherein the piston rod comprises the abutment portion of the piston.

Preferably the piston comprises a lever portion which is accessible from an exterior of the applicator.

Preferably the lever portion allows a user to move the piston independently of the piston actuating means.

Preferably the first fluid enters the at least one barrel at or adjacent a first end of the at least one barrel, and the first fluid exits the at least one barrel at or adjacent the first end.

Preferably the at least one fluid inlet valve is adjacent one end of the at least one barrel, and the at least one fluid outlet valve is adjacent the same end of the at least one barrel.

Preferably the at least one fluid inlet valve and the at least one fluid outlet valve are adjacent the first end, and an outlet, wherein the outlet is at a second end of the at least one barrel, opposite to the first end.

The applicator further comprises,
- a second barrel,
- a second piston moveable between a first position and a second position within the second barrel, wherein, in use, movement of the second piston towards the first position can draw a second fluid into the second barrel, and movement of the piston towards the second position can forces the second fluid out of the second barrel,
- at least one second fluid inlet to allow the second fluid to flow into the second barrel, and
- second biasing means to bias the second piston toward the first position,
wherein the piston actuating means is operable to move both the first and second pistons towards the second position to dispense the first fluid and the second fluid from the applicator.

The applicator includes at least one second outlet valve in fluid contact with the second barrel to allow the second fluid to flow out of the second barrel.

The applicator includes a second dosage control part to abut a second abutment portion which in turn defines the first position of the second piston.

Preferably the second piston comprises a second piston head and a second piston rod connected to or integral with the second piston head, wherein the second piston rod comprises the second abutment portion of the second piston.

The second dosage control part is slideable along an axis which is substantially parallel to a longitudinal axis of the first and second barrels.

The applicator further comprises a second lever portion which allows a user to move the second piston independently of the piston actuating means, and independently of the first piston.

The non-therapeutic **method of operating an applicator** comprises or includes the steps of
Fluidly connecting supply of a first fluid to a manifold of the applicator,
Adjusting a dosage of a first piston with a first barrel of the applicator characterised by using a first dosage control part, where the first dosage control part is slidingly lockable along a major axis of the first barrel, the first dosage control part abutting a first abutment portion on the first piston which in turn varies a first position of the first piston and therefore the dosage of the first fluid,
Priming the first barrel using a first lever portion,
Forcing a first fluid from the first barrel by moving the first piston from the first position to a second position within the barrel, by use of a piston actuating means which, the first fluid moving from the first barrel via at least one fluid outlet valve,
Drawing the first fluid from the supply into the first barrel by the first piston moving from the second position back to the first position, the first fluid moving in via a first fluid inlet valve, the movement from the first position to the second position being biased,
wherein a user may set the dosage of the applicator via the first dosage control part.

Further aspects of the invention, which should be considered in all its novel aspects, will become apparent from the following description given by way of example of possible embodiments of the invention.

### Brief Description of the Drawings

- **Figure 1**: is a perspective view of an applicator according to one embodiment of the present invention,
- **Figure 2**: is a side view of the applicator of Figure 1,
- **Figure 3**: is a perspective view of the applicator of Figure 1, with the housing and first handle removed,
- **Figure 4**: is a top view of the applicator of Figure 1, with the housing and first handle removed,
- **Figure 5**: is a top view of the relative layout of the pistons and dosage control parts of the applicator of Figure 1,
- **Figure 6**: is a perspective view of the second handle, pistons and dosage control parts of the applicator of Figure 1, with the second dosage control part shown in partially exploded form,
- **Figure** 7: shows the relative layout of the second handle, conduits and valves of the applicator of Figure 1,
- **Figure 8**: is a partially exploded perspective view of the first dosage control part,
- **Figure 9**: shows a side view of a further embodiment of the present invention, where the manifold is located at the top rear of the body,
- **Figure 10**: shows the rear view of the embodiment of Figure 9,
- **Figure 11**: shows the plan view of the embodiment of Figure 9,
- **Figure 12**: shows a rear isometric view of the embodiment in Figure 9,
- **Figure 13**: shows an exploded view of the embodiment of Figure 9,
- **Figure 14**: shows the rear end of the exploded view of Figure 13,
- **Figure 15**: shows the middle portion of the exploded view of Figure 13, and
- **Figure 16**: shows the front end of the exploded view of Figure 13.

### Brief Description of Preferred Embodiments

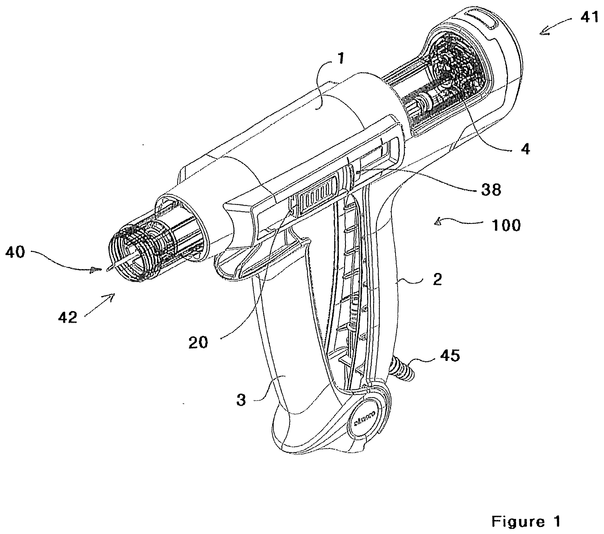

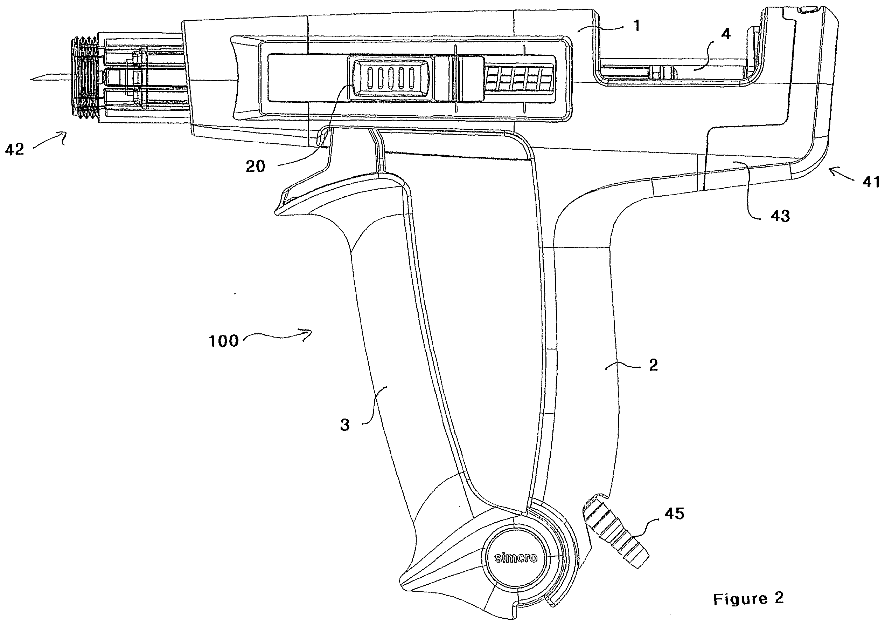

Referring first to Figures 1-4, an applicator according to one embodiment of the present invention is generally referenced by arrow 100.

The applicator described has two barrels and associated components to deliver two fluids, or a greater volume of a single fluid from the two barrels. A single barrel applicator, though not shown, is not an embodiment of the present invention. Such a single barrel applicator may be buildable from the dual barrel applicator shown, for example by disassembly and replacing the barrel, manifold and associated components with a single barrel. In this way it may be made with the same body, and second handle, outlet but have a single barrel and piston and adjusters so that where possible common parts may be used.

Alternatively all, or a majority of, the components may be specific to the single barrel applicator.

Also an applicator with more than 2 barrels, though not shown, is also disclosed. A person skilled in the art would understand how such an applicator could have three or more barrels arranged equally about the longitudinal centre line, each with its own piston, valving, adjustment and priming levers.

The applicator 100 comprises a housing 1 which is connected to or integral with a first handle 2, and has a first end 41 and second end 42. A second handle 3 is pivotally connected at a lower end to the first handle 2. In use, movement of the second handle 3 relative to the first handle 2, and thus the body or housing 1 activates the applicator 100, as is described further below, to deliver fluid(s) from an outlet 40 at the second end 42. The second handle 3 drives a piston, one per barrel 4, to draw fluid into the barrel, and expel it out again. The dosage of the barrel can be adjusted using dosage control parts 20, and in the case of two barrels second dosage control part 21. To prime the barrel, or barrels there is a lever portion 38, and 39, to fill the barrel(s) with fluid. This is so there is minimal wastage with two or more barrels.

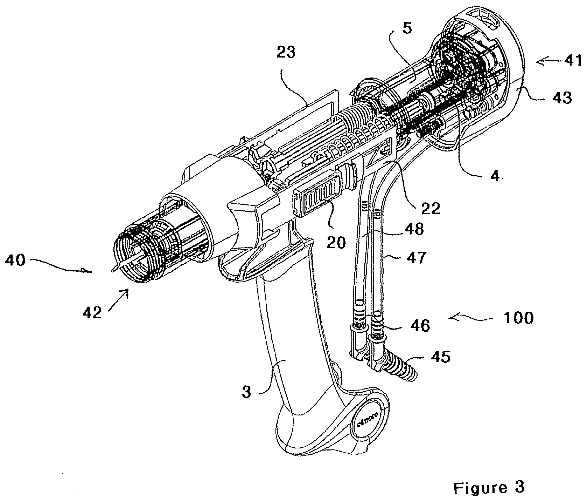

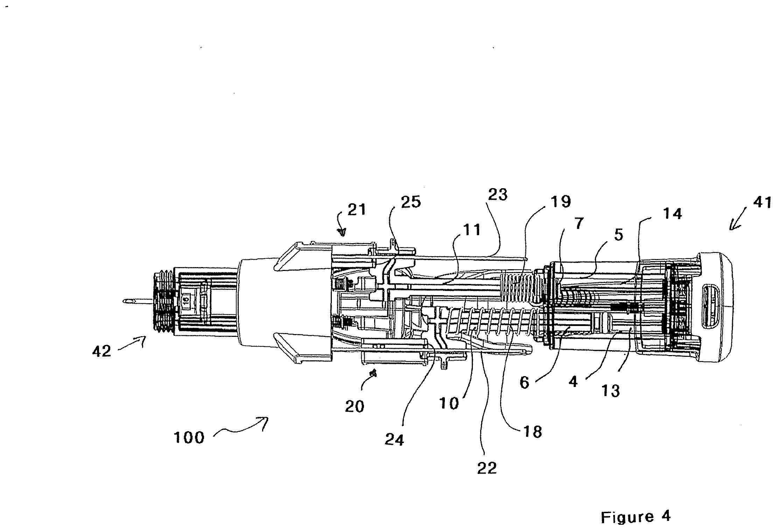

Referring next to Figures 3 and 4 in particular, the applicator 100 has two barrels 4, 5 which are mounted to or integral with the housing 1 (not shown here). The barrels 4, 5 are preferably adjacent to and parallel with each other. In the embodiment shown the barrels 4, 5 have the same diameter. However, in many embodiments the first barrel 4 will have a different diameter to the second barrel 5. The barrels 4, 5 shown in Figures 3 and 4 are substantially circular in cross-section. However, in some embodiments (not shown) the barrels may be substantially "D" shaped in cross-section, in order to reduce the overall width of the applicator.

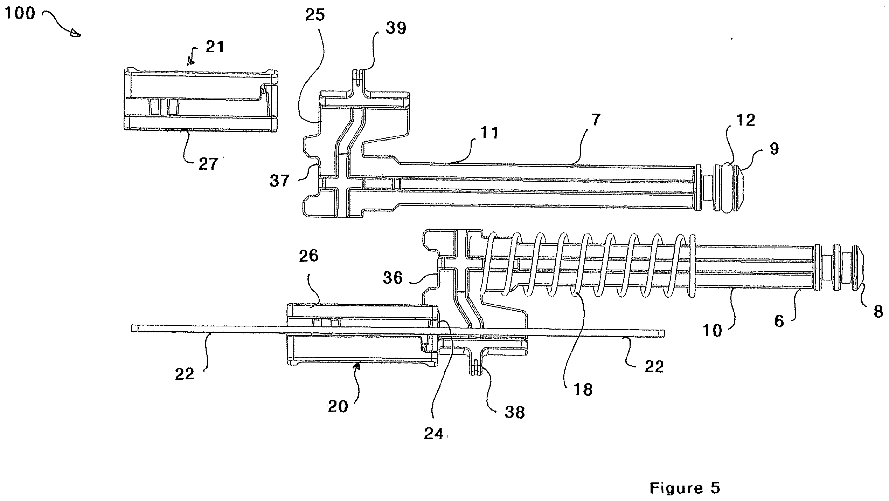

As shown in Figures 4 to 6, each barrel 4, 5 is provided with a respective piston 6, 7, the pistons 6, 7 each comprising a head 8, 9 which is connected to or integral with a respective piston rod 10, 11. The piston heads 8, 9 are provided with suitable seals 12 (the seal from the first piston 6 is omitted in Figure 5) to seal against an inner wall of the respective barrel 4, 5 to thereby form a chamber 13, 14. As will be familiar to those skilled in the art, the volume of the chambers 13, 14 varies as the pistons 6, 7 are moved within the barrels 4, 5 between a variable first position and a second position, the volume being greater when the pistons 6, 7 are in the first position. This is shown for example in Figure 4, where first piston 6 is adjusted (explained below) so that its first position, which is variable, is further into its travel when compared to the second piston 7 which is adjusted (explained below) so that its first position, which is also variable, is less further into its travel.

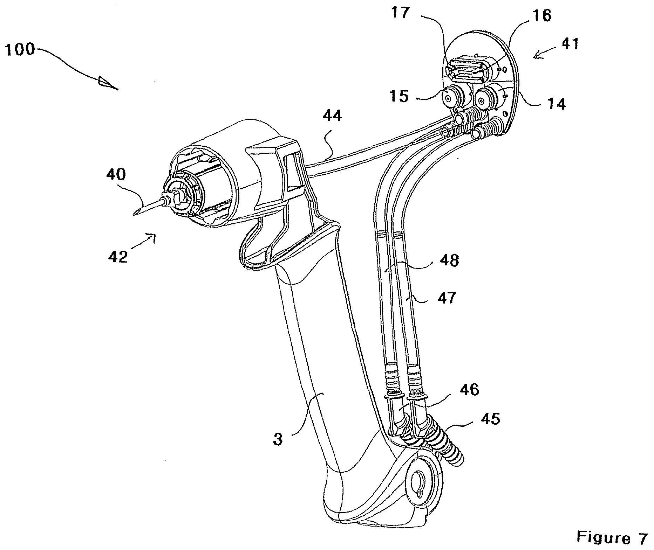

Referring next to Figures 4 and 7, suitable inlet valves 14, 15 and outlet valves 16, 17 are provided for each barrel 4, 5. The inlet and outlet valves are one-way valves. In the embodiment shown the inlet and outlet valves are provided at one end of the respective barrels 4, 5, but in other embodiments (not shown) the inlet and outlet valves may be provided in conduits which are in fluid communication with the barrels 4, 5. In another embodiment (not shown) the barrels 4, 5 may share a common outlet valve or a plurality of common outlet valves.

Movement of the pistons 6, 7 towards their respective first positions (to the left when seen from above in Figures 4 through 7) draws fluid into the respective barrels through the respective inlet valves 14, 15, and movement of the pistons 6,7 towards the second position (to the right when seen from above in Figures 4 through 7) forces fluid from the barrels through the outlet valves 16, 17.

Referring back to Figures 4 to 6, each piston 5, 6 is provided with a biasing means, typically a spring 18, 19 (only one spring shown in Figure 5), to bias the piston 4, 5 towards the first position.

The travel of the piston 4, 5 under the influence of the biasing means 18, 19 (and therefor the maximum volume of each chamber) is limited by respective dosage control parts 20, 21. The dosage control parts 20, 21 thereby define the first position for the pistons. In the embodiment shown each dosage control part 20, 21 is an assembly and is provided as an adjustable stop which is slideably engaged with a respective slotted component 22, 23 which is attached to or integral with the body 1.

Referring next to Figures 4 to 6, each piston rod 10, 11 has an abutment portion 24, 25 which abuts the respective dosage control part 20, 21. Note that in Figure 5, dosage control part 21 is shown spaced apart from abutment portion 25 for clarity.

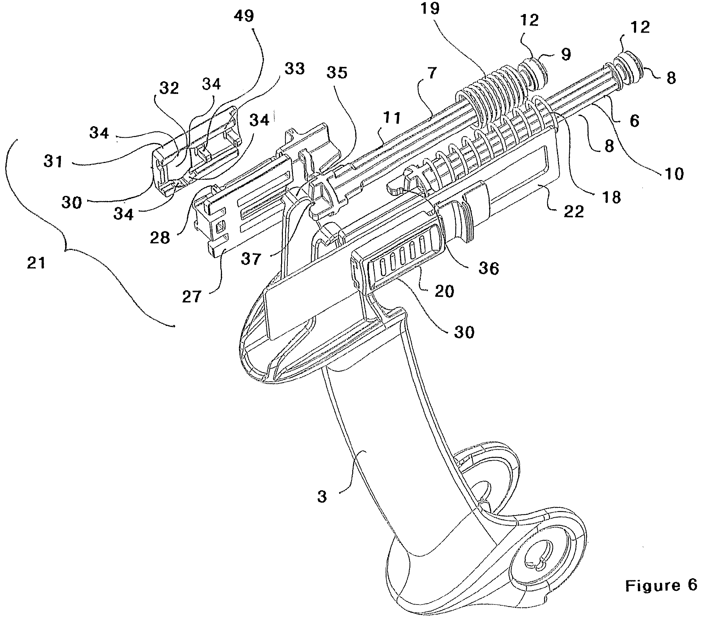

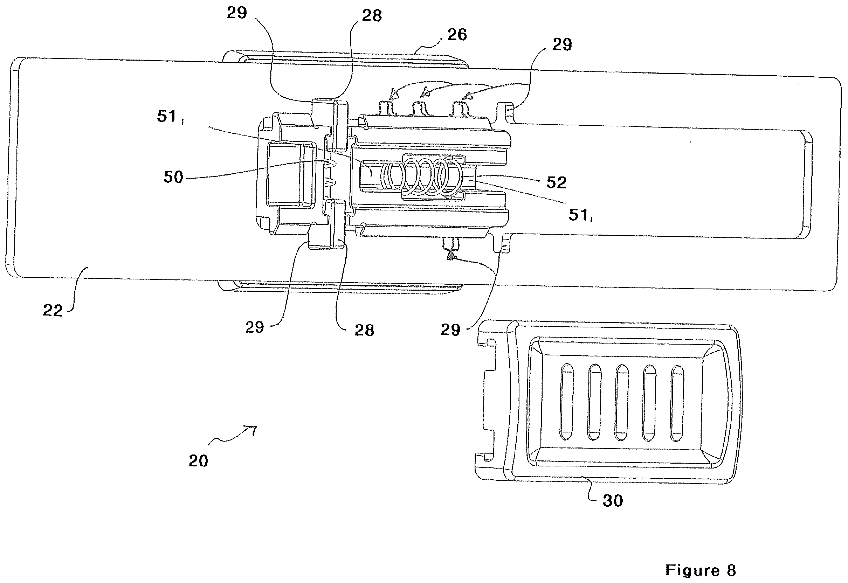

Referring next to Figures 6 and 8, in the embodiment shown the dosage control parts 20, 21 comprise in the embodiment shown an assembly of a dosage control part body 26, 27, each provided with at least one, and more preferably a pair of moveable locking tabs 28 which engage complementary notches 29 provided in the sides of the slot of the slotted component 22, 23 (best seen in Figure 8). The tabs 28 are outwardly biased, using a tab spring 50. This tab spring (or springs if needed) can act on a suitable part of the dosage control part body, or for example where there are two locking tabs as shown in Figure 8, may engage on each other to bias the tabs outwardly (vertically in the case of Figure 8)

The locking tabs 28 are moved inwardly (described below) to disengage the notches 29. The slotted component is shown in Figure 8 as having eight notches 29. However, in other preferred forms as needed the notches 29 are anywhere along the length of the slotted component 28. This will provide varying levels of adjustment and therefore differing first positions to provide varying dosages as needed. In other form, where there are two locking tabs 28 present, the notches 29 may be staggered on either side to provide additional resolution to the adjustable first position to in turn provide a greater variety of doses. In this embodiment each locking tab may alternatively engage a notch on one side, and then the other locking tab will engage another notch further along on the other side. Suitable markings are provided on the dosage control part and the body, for example an indicator mark on the dosage control part, for example the button 31, and dosage volume markings (for example in millilitres) on the body, or vice versa.

The slotted components 22, 23 and locking tabs 28 are in the preferred embodiment made from a tough resilient material to withstand repeated adjustments and repeated stops and starts of the respective piston movements. In one form they may be made of metal, preferably no-ferrous, such as stainless steel.

Shown in Figure 6 each dosage control part 20, 21 is provided with an exterior button 30, 31 which has a recess 32 on an inward face 33 which is provided with angled sides or ramps 34. The ramps as shown in Figure 6, taper inwardly from the interior toward the button 30, 31 exterior. This is such that the angled sides 34 of the recess 32 move the tabs 28 inwardly and therefore out of engagement with the respective notches 29 when the button 30, 31 is moved sideways that is, inward, relative to the body. In an alternative embodiment (not shown) the slot may not be provided with notches. In this embodiment each dosage control part may be held in a required position by alternative means, such as friction.

The button is further biased outwardly from the body 1. Present on the interior side of the dosage control part, and in this embodiment shown the interior side of the button 30 and 31, are ramped extensions 51 (shown in Figure 6). These taper inward from the interior side of the button, towards its exterior. Residing between them is a button spring 52 (seen in Figure 8), which bears on the ramped extensions 51 to bias the button outwardly.

Referring next to Figures 1, 5, 6 and 7, in use, fluid is expressed from the applicator 100 by squeezing the first and second handles 2, 3 towards each other. This will move the second handle 3 along the body 1 to actuate the pistons 6 and 7. As is best seen in Figures 5 and 6, movement of the second handle 3 causes an engagement portion 35 of the handle (typically provided at an opposite end of the handle 3 to the pivotal connection with the first handle 2) to move into engagement with a second abutment portion 36, 37 of each piston 6, 7. Shown in Figure 5 the abutment portions 36, 37 are at differing location due to the dosage they are adjusted to.

Depending on the relative settings of the dosage control parts 20, 21, the engagement of the engagement portion 35 with the second abutment portions 36, 37 therefore may not occur simultaneously. For example, in the embodiment shown in Figure 5 and 6 the engagement of the engagement portion 35 with second abutment portion 37 of piston 7 will occur first, and fluid will begin to be expressed from the barrel 5 associated with piston 7 before any fluid is expressed from the barrel 4 associated with piston 6. In some embodiments it may be possible to adjust the dosage control parts 26, 27 such that substantially no fluid is expressed from the respective barrel. In this way a dual barrel applicator may be used to express only one fluid, if required.

As can be seen from Figures 4 and 6, in contrast with the dosage control mechanisms of the prior art, the engagement portion 35 is able to move past the dosage control parts 20, 21 during actuation of the pistons 6, 7. This allows the length of the piston rods 10, 11 (and therefore the entire applicator) to be reduced, as otherwise the piston rods 10, 11 would need to be longer in order to allow each piston to move over its full stroke.

As best seen in Figure 5, one or both of the piston rods 10, 11 are provided with a lever portion 38, 39 which extends though the slot and is accessible from the exterior of the applicator 100. This lever portion 38, 39 can be used to actuate the respective piston 6, 7 independently from the handle 3 or the other piston. This is useful where the applicator 100 must be primed before use. To prime the applicator 100 the handle 3 may be operated until one of the barrels 4, 5 is full. The barrels 4, 5 may be transparent or translucent to allow the user to see when this has occurred. When the first barrel to fill has filled, the operator may use the lever portion 38, 39 of the piston associated with the other barrel to actuate that piston until the second barrel is full. This allows the device to be primed with minimum waste.

Referring next to Figures 1, 3 and 7, in preferred embodiments the inlet valves 14, 15 and outlet valves 16, 17 for each barrel 4, 5 are provided at a first end 41 of the applicator, and an outlet 40 is provided at an opposite second end 42 of the applicator. In preferred embodiments each barrel 4, 5 has an expanded portion or a manifold portion at one end to allow the inlet and outlet valves to be provided at the same end of the barrel. Grouping of the inlet and outlet valves together in this way allows easy access to all of the valves by removing a single cover 43, rather than requiring more substantial disassembly of the applicator. In preferred embodiments the outlet valves 16, 17 feed a manifold (not shown) which combines the fluids from the barrels 4, 5 before a single outlet conduit 44 feeds the combined fluid to the outlet 40. If required the manifold and/or outlet conduit 44 may be provided with suitable vanes or other formations (not shown) to ensure that the fluids from the barrels 4, 5 are properly mixed together prior to being expressed through the outlet. In other embodiments (not shown) the outlet fluids may be prevented from mixing until reaching the front or first end 42 of the applicator 100 by providing separate outlet conduits (a duplication of conduit 42) from each barrel to the outlet 44 - the dual conduits joining using a "Y" join (not shown) at or near the outlet. This embodiment reduces the mixed volume of the two products prior to application. Minimal mixed product may be the preferred practice, depending on the specific products to be combined.

The inlet valves 14, 15 are connected to respective inlet connections 45, 46 by inlet conduits 47, 48. In the embodiment shown the inlet connections are 45, 46 are provided at the base of the first handle 2. In another embodiment (not shown) the inlet connections 45, 46 may be provided at the second end 42 of the applicator.

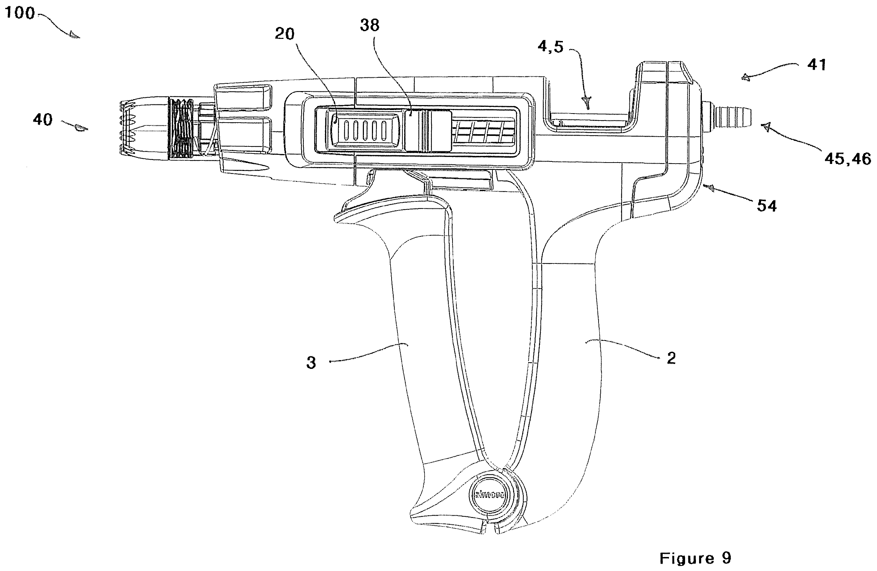

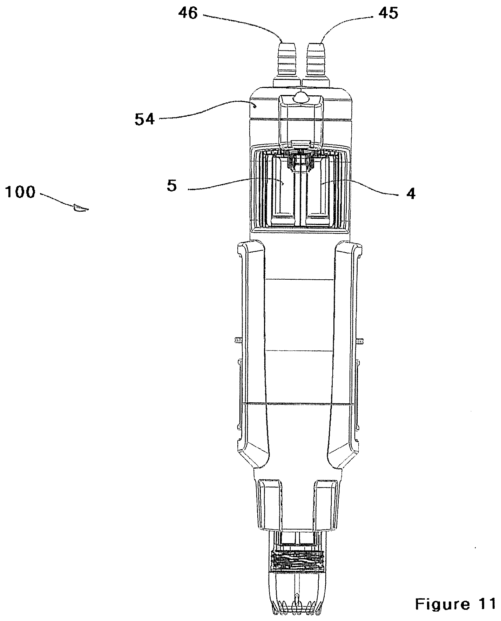

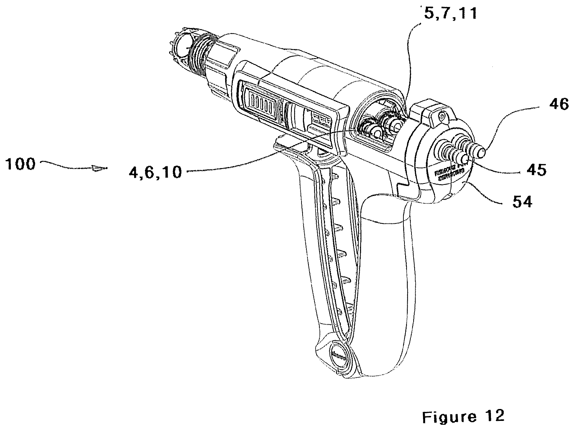

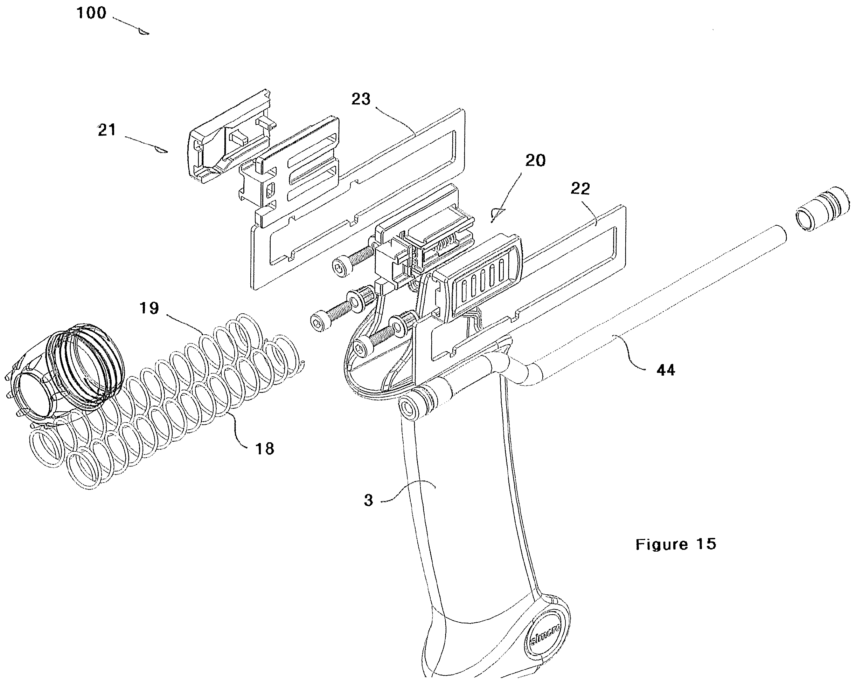

In another embodiment, shown in Figures 9 through 16 the inlet connections 45, 46 may be an integral part of a manifold provided adjacent the barrels at the first end 41. Reference numerals in these Figures, where they are the same, are for the same features.

Again, the applicator 100 comprises a housing 1 which is connected to or integral with a first handle 2, and has a first end 41 and second end 42. A second handle 3. Shown in the first position here, is pivotally connected at a lower end to the first handle 2. A manifold 54 contains the connections 45, 46 to the fluid supply (not shown), and the inlet and outlet valves (not shown) for the barrel 4, 5. In use, movement of the second handle 3 relative to the first handle 2, and thus the body or housing 1 activates the applicator 100, as is described above, to deliver fluid(s) from an outlet 40 at the second end 42. The second handle 3 drives a piston, one per barrel 4, to draw fluid into the barrel, and expel it out again. The dosage of the barrels can be adjusted using dosage control parts 20, 21. To prime the barrels there is a lever portion 38, and 39, to fill the barrel(s) with fluid. This is so there is minimal wastage when there are two or more barrels.

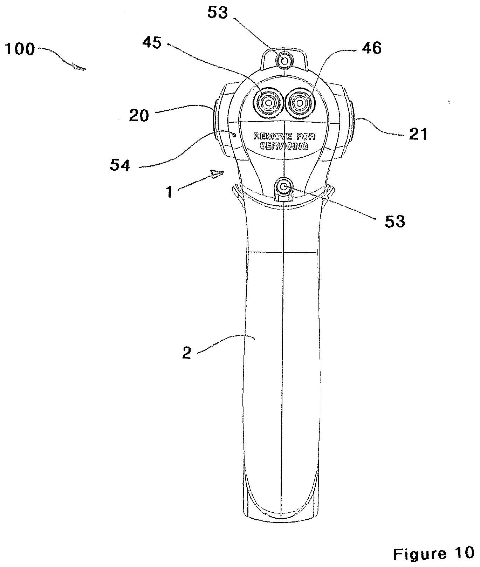

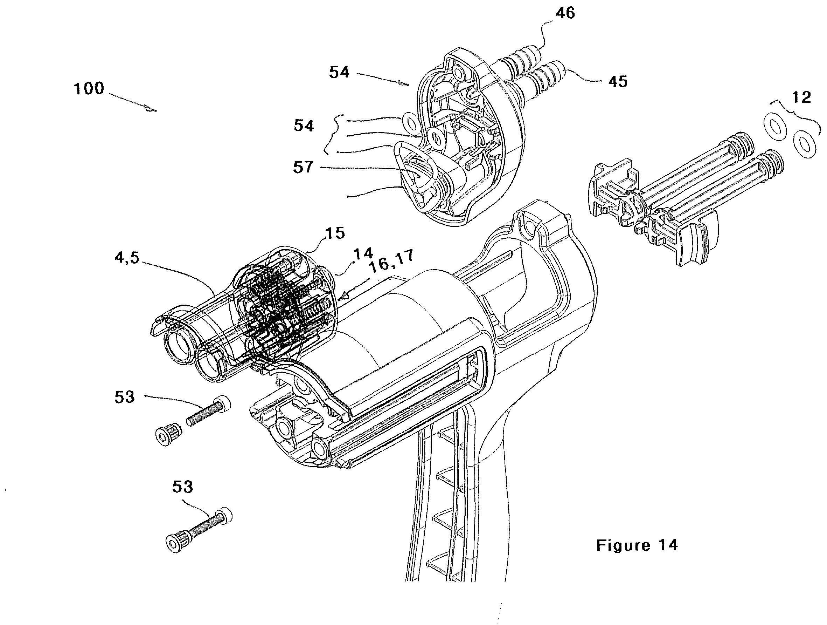

The manifold 54 is better shown in Figures 10 through 14. The manifold 54 can be attached in any re-connectable way, and in the preferred form uses a pair of fasteners 53 as shown in Figures 10, 13 and 14. Removing the fasteners allows the manifold to be removed from the first end 41 of the applicator 100, as shown in Figure 14. Removing the manifold 54 allows access to the inlet valves 14, 15 (in this embodiment they are contained as part of the barrel 4,5 assembly), outlet valves 16, 17 and their respective seals 54. The mixing manifold 57 in this case is shown immediately downstream of the outlet valves 16, 17 and connects to the outlet conduit 44. Once the manifold 54 is removed, the barrel assembly 4, 5 can be extracted from the first end.

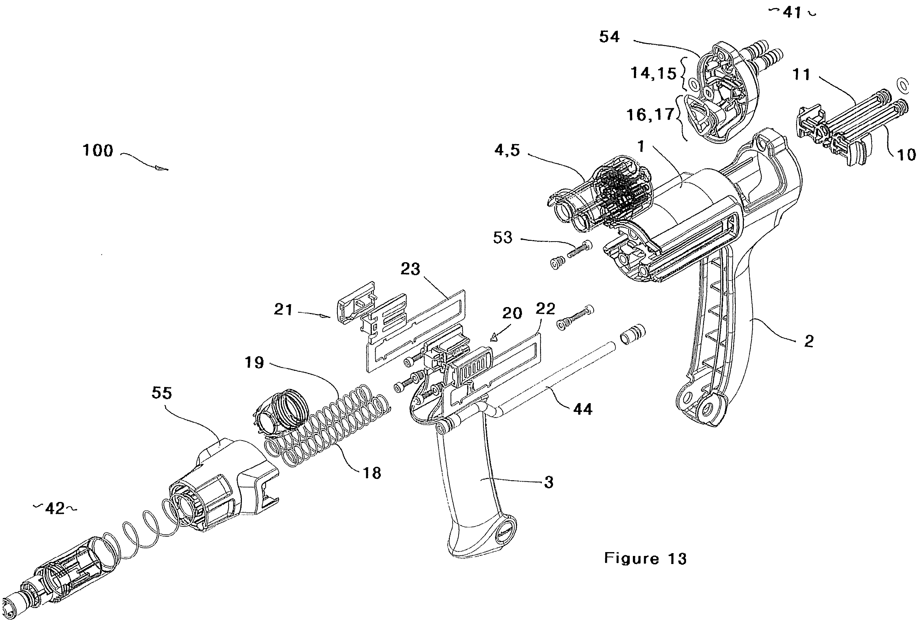

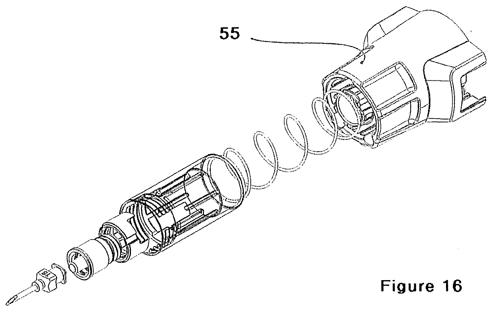

Likewise a housing front 55, shown in Figures 13 and 16 can be removed (again here with fasteners) from the body 1. This, in turn, allows for extraction of the piston rods 10, 11 and their pistons, seals, springs and abutment portions. Likewise the dosage control parts, and second handle 3 can be removed. In this way the entire applicator 100 can easily be assembled with minimal separate parts, to allow cleaning, removing of blockages and repair or replacement.

The applicator shown in the Figures and described above is configured as an injector. However, it will be appreciated that other embodiments may be adapted for many other uses, including "pour-on" applicators, oral applicators, or nasal infusion applicators.

In some embodiments (not shown) the applicator may be configured as a "bottle mount" style injector, with a bottle mount fitting provided at the first end of the applicator. In this embodiment the (or each) inlet valve is provided adjacent the first end of the barrel. As is typical with bottle mount injectors, a short conduit portion may connect the inlet valve with an aperture in a side wall of the barrel.

While the invention has been particularly described with reference to a dual barrel applicator, in other embodiments not part of the present invention may be configured as a single barrel applicator. The applicator may be configured with more than two barrels (for example three barrels).

Throughout the claims, the words "comprise", "comprising", and the like, are to be construed in an inclusive sense as opposed to an exclusive or exhaustive sense, that is to say, in the sense of "including, but not limited to".

Although this invention has been described by way of example and with reference to possible embodiments thereof, it is to be understood that modifications or improvements may be made thereto without departing from the scope of the claims.

## Claims

1. An **applicator** (100) comprising:
- two or more barrels comprising a first barrel (4);
- a piston (6) moveable within the first barrel (4) between a variable first position and a second position, wherein, in use, movement of the piston (6) towards the first position can draw a first fluid into the first barrel (4), and movement of the piston (6) towards the second position can force the first fluid out of the first barrel (4);
- two or more fluid inlet valves (14, 15) comprising a fluid inlet valve (14) to allow the fluid to flow into the first barrel (4) at least under action of the piston (6), ;
- two or more fluid outlet valves (16, 17) comprising a fluid outlet valve (16) to allow the first fluid to flow out of the first barrel (4) at least under action of the piston (6),;
- biasing means (18) to bias the piston (6) toward the first position,;
**characterised in that**:
said two or more barrels comprise a second barrel (5);
said two or more fluid inlet valves (14, 15) comprise a second fluid inlet valve (15) to allow the second fluid to flow into the second barrel (5);
said two or more fluid outlet valves (16, 17) comprise a second fluid outlet valve (17) to allow the second fluid to flow out of the second barrel (5); wherein the applicator further includes
- a second piston (7) moveable between a first position and a second position within the second barrel (5), wherein, in use, movement of the second piston (7) towards the first position can draw a second fluid into the second barrel (5), and movement of the second piston (7) towards the second position can force the second fluid out of the second barrel (5);
- second biasing means (19) to bias the second piston (7) towards the first position;
- a dosage control part (20) that slides linearly on a longitudinal axis of the first barrel (4), and can lock in any one of a plurality of notches (29) that each correspond to a dosage mark to abut an abutment portion (24) of the piston which in turn defines the variable first position of the piston (6) and therefore a dosage of the first fluid;
- a second dosage control part (21) that slides along an axis which is parallel to the longitudinal axis of the first barrel, and can lock in any one of a plurality of notches that each correspond to a dosage mark to abut a second abutment portion (25) of the second piston which in turn defines the variable first position of the second piston and therefore a dosage of the second fluid; and
- piston actuating means operable to move the piston (6) and the second piston (7) towards their respective second positions to dispense the first fluid and the second fluid from the applicator (100),
wherein a user may set the dosage of each of the first barrel and the second barrel via the dosage control part (20) and second dosage control part (21), and deliver a dosage of the first fluid and the second fluid by the piston actuating means.

2. The applicator (100) of claim 1, wherein the abutment portion (24) of the piston is on, or connected to, the piston (6), and wherein the second abutment portion (25) of the second piston is on, or connected to, the second piston (7).

3. The applicator (100) of either claim 1 or 2, wherein: (i) the piston (6) comprises a piston head (8) and a piston rod (10) connected to or integral with the piston head (8), wherein the piston rod (10) comprises the abutment portion (24) of the piston (6); and (ii) the second piston (7) comprises a second piston head (9) and a second piston rod (11) connected to or integral with the second piston head (9), wherein the second piston rod (11) comprises the abutment portion (25) of the second piston (7).

4. The applicator of any one of claims 1 to 3, wherein the piston (6) comprises a lever portion (38) which is accessible from an exterior of the applicator (100), and wherein the second piston (7) comprises a second lever portion (39) which is accessible from an exterior of the applicator (100).

5. The applicator (100) of claim 4, wherein the second lever portion (39) allows a user to move the second piston (7) independently of the first piston (6).

6. The applicator (100) of claim 5, wherein the lever portion (38) and the second lever portion (39) allow a user to move each piston (6, 7) independently of the piston actuating means.

7. The applicator (100) of any one of claims 1 to 6, wherein the first fluid enters the first barrel (4) and the second fluid enters the second barrel (5) at or adjacent a first end of the two or more barrels (4, 5), and the first fluid exits the first barrel (4) and the second fluid exits the second barrel (5) at or adjacent the first end.

8. The applicator (100) of claim 7 wherein the two or more fluid inlet valves (14, 15) are adjacent one end of the two or more barrels (4, 5), and the two or more fluid outlet valves (16, 17) are adjacent the same end of the two or more barrels (4, 5).

9. The applicator (100) of either claim 7 or claim 8, wherein the two or more fluid inlet valves (14, 15) and the two or more fluid outlet valves (16, 17) are adjacent the first end, and wherein the applicator further comprises an outlet (40), wherein the outlet (40) is at a second end of the two or more barrels (4, 5), opposite to the first end.

10. A non-therapeutic **method of operating an applicator** (100), comprising or including the steps of
- fluidly connecting supply of a first fluid to a manifold (54) of thtor (100),
- priming a first barrel (4) of the applicator using a first lever portion (38),
- forcing a first fluid from the first barrel (4) by moving a first piston (6) from a first position to a second position within the first barrel (4), by use of a piston actuating means which, the first fluid moving from the first barrel (4) via at least one fluid outlet valve (16),
- drawing the first fluid from the supply into the first barrel (4) by the first piston (6) moving from the second position back to the first position, the first fluid moving in via a first fluid inlet valve (14), the movement from the first position to the second position being biased,
wherein a user may set the dosage of the applicator (100) via a first dosage control part (20),; and
**characterized by** further comprising the steps of,
- before priming the first barrel (4), adjusting a dosage of the first piston (6) within the first barrel (4) by using the first dosage control part (20), where the first dosage control part is slidingly lockable along a major axis of the first barrel (4) in any one of a plurality of notches that each correspond to a dosage mark, the first dosage control part (20) abutting a first abutment portion (24) on the first piston (6) which in turn varies a first position of the first piston (6) and therefore the dosage of the first fluid, and
-fluidly connecting a supply of a second fluid to the manifold (54) of the applicator (100),
- adjusting a dosage of a second piston (7) within a second barrel (5) of the applicator (100) using a second dosage control part (27), where the second dosage control part (27) is slidingly lockable along a major axis of the second barrel (5), the second dosage control part abutting a second abutment portion (36, 37) on the second piston (7) which in turn varies a first position of the second piston (7) and therefore the dosage of the second fluid,
- priming the second barrel (5) using a second lever portion (39),
- forcing a second fluid from the second barrel (5) by moving the second piston (7) from the first position to a second position within the barrel, by use of a piston actuating means which, the second fluid moving from the second barrel via at least one second fluid outlet valve,
- drawing the second fluid from the second supply into the second barrel (5) by the second piston moving from the second position back to the first position, the second fluid moving in via a second first fluid inlet valve, the movement from the first position to the second position being biased,
wherein a user may set the dosage of the applicator (100) via the second dosage control part (27).

## Patentansprüche

1. Applikator (100), umfassend:
- zwei oder mehr Zylinder, umfassend einen ersten Zylinder (4);
- einen Kolben (6), der innerhalb des ersten Zylinders (4) zwischen einer variablen ersten Position und einer zweiten Position bewegbar ist, wobei im Betrieb eine Bewegung des Kolbens (6) zu der ersten Position ein erstes Fluid in den ersten Zylinder (4) saugen kann und eine Bewegung des Kolbens (6) zu der zweiten Position das erste Fluid aus dem ersten Zylinder (4) herausdrücken kann;
- zwei oder mehr Fluideinlassventile (14, 15), umfassend ein Fluideinlassventil (14), um das Fließen des Fluids in den ersten Zylinder (4) zumindest unter Einwirkung des Kolbens (6) zu ermöglichen;
- zwei oder mehr Fluidauslassventile (16, 17), umfassend ein Fluidauslassventil (16), um das Fließen des ersten Fluids aus dem ersten Zylinder (4) heraus zumindest unter Einwirkung des Kolbens (6) zu ermöglichen;
- Vorspannmittel (18), um den Kolben (6) in Richtung der ersten Position vorzuspannen;
**dadurch gekennzeichnet, dass**:
die zwei oder mehr Zylinder einen zweiten Zylinder (5) umfassen;
die zwei oder mehr Fluideinlassventile (14, 15) ein zweites Fluideinlassventil (15) umfassen, um das Fließen des zweiten Fluids in den zweiten Zylinder (5) zu ermöglichen;
die zwei oder mehr Fluidauslassventile (16, 17) ein zweites Fluidauslassventil (17) umfassen, um das Fließen des zweiten Fluids aus dem zweiten Zylinder (5) heraus zu ermöglichen; wobei der Applikator ferner umfasst
- einen zweiten Kolben (7), der innerhalb des zweiten Zylinders (5) zwischen einer ersten Position und einer zweiten Position bewegbar ist, wobei im Betrieb eine Bewegung des zweiten Kolbens (7) zu der ersten Position ein zweites Fluid in den zweiten Zylinder (5) saugen kann und eine Bewegung des zweiten Kolbens (7) in Richtung der zweiten Position das zweite Fluid aus dem zweiten Zylinder (5) herausdrücken kann;
- ein zweites Vorspannmittel (19), um den zweiten Kolben (7) in Richtung der ersten Position vorzuspannen;
- ein Dosiersteuerteil (20), das linear auf einer Längsachse des ersten Zylinders (4) gleitet und in einer beliebigen einer Vielzahl von Kerben (29) arretiert werden kann, die jeweils einer Dosiermarkierung entsprechen, um an einem Anschlagabschnitt (24) des Kolbens anzuliegen, der wiederum die variable erste Position des Kolbens (6) und damit eine Dosierung der ersten Flüssigkeit definiert;
- ein zweites Dosiersteuerteil (21), das entlang einer Achse gleitet, die parallel zur Längsachse des ersten Zylinders verläuft, und in einer beliebigen von mehreren Kerben arretiert werden kann, die jeweils einer Dosiermarkierung entsprechen, um an einem zweiten Anschlagabschnitt (25) des zweiten Kolbens anzuliegen, der wiederum die variable erste Position des zweiten Kolbens und damit eine Dosierung der zweiten Flüssigkeit definiert; und
- eine Kolbenbetätigungseinrichtung, die betätigbar ist, um den Kolben (6) und den zweiten Kolben (7) in Richtung ihrer jeweiligen zweiten Positionen zu bewegen, um das erste Fluid und das zweite Fluid aus dem Applikator (100) abzugeben,
wobei ein Benutzer die Dosierung sowohl des ersten Zylinders als auch des zweiten Zylinders über das Dosiersteuerteil (20) und das zweite Dosiersteuerteil (21) einstellen kann, und eine Dosierung der ersten Flüssigkeit und der zweiten Flüssigkeit durch die Kolbenbetätigungseinrichtung abgeben.

2. Applikator (100) nach Anspruch 1, wobei sich der Anschlagabschnitt (24) des Kolbens am Kolben (6) befindet oder mit diesem verbunden ist und wobei sich der zweite Anschlagabschnitt (25) des zweiten Kolbens am zweiten Kolben (7) befindet oder mit diesem verbunden ist.

3. Applikator (100) nach Anspruch 1 oder 2, wobei: (i) der Kolben (6) einen Kolbenkopf (8) und eine mit dem Kolbenkopf (8) verbundene oder mit diesem einstückige Kolbenstange (10) umfasst, wobei die Kolbenstange (10) den Anschlagabschnitt (24) des Kolbens (6) bildet; und (ii) der zweite Kolben (7) einen zweiten Kolbenkopf (9) und eine zweite Kolbenstange (11) umfasst, die mit dem zweiten Kolbenkopf (9) verbunden oder einstückig mit diesem ausgebildet ist, wobei die zweite Kolbenstange (11) den Anschlagabschnitt (25) des zweiten Kolbens (7) umfasst.

4. Applikator nach einem der Ansprüche 1 bis 3, wobei der Kolben (6) einen Hebelabschnitt (38) aufweist, der von der Außenseite des Applikators (100) zugänglich ist, und wobei der zweite Kolben (7) einen zweiten Hebelabschnitt (39) aufweist, der von der Außenseite des Applikators (100) zugänglich ist.

5. Applikator (100) nach Anspruch 4, wobei der zweite Hebelabschnitt (39) es einem Benutzer ermöglicht, den zweiten Kolben (7) unabhängig vom ersten Kolben (6) zu bewegen.

6. Applikator (100) nach Anspruch 5, wobei der Hebelabschnitt (38) und der zweite Hebelabschnitt (39) es einem Benutzer ermöglichen, jeden Kolben (6, 7) unabhängig von der Kolbenbetätigungseinrichtung zu bewegen.

7. Applikator (100) nach einem der Ansprüche 1 bis 6, wobei das erste Fluid in den ersten Zylinder (4) und das zweite Fluid in den zweiten Zylinder (5) an oder nahe einem ersten Ende der zwei oder mehr Zylinder (4, 5) eintritt und das erste Fluid aus dem ersten Zylinder (4) austritt und das zweite Fluid aus dem zweiten Zylinder (5) austritt an oder nahe dem ersten Ende.

8. Applikator (100) nach Anspruch 7, wobei die zwei oder mehr Fluideinlassventile (14, 15) das einem Ende der zwei oder mehr Zylinder (4, 5) angrenzen sind und die zwei oder mehr Fluidauslassventile (16, 17) an demselben Ende der zwei oder mehr Zylinder (4, 5) angrenzen.

9. Applikator (100) nach Anspruch 7 oder Anspruch 8, wobei die zwei oder mehr Fluideinlassventile (14, 15) und die zwei oder mehr Fluidauslassventile (16, 17) an das erste Ende angrenzen, und wobei der Applikator ferner einen Auslass (40) umfasst, wobei sich der Auslass (40) an einem zweiten Ende der zwei oder mehr Zylinder (4, 5) befindet, das dem ersten Ende gegenüberliegt.

10. Nicht-therapeutisches Verfahren zum Betreiben eines Applikators (100), umfassend oder beinhaltend die folgenden Schritte:
- fluidisches Verbinden der Zufuhr eines ersten Fluids mit einem Verteiler (54) des Applikators (100),
- Vorfüllen eines ersten Zylinders (4) des Applikators unter Verwendung eines ersten Hebelabschnitts (38),
- Ausdrücken eines ersten Fluids aus dem ersten Zylinder (4) durch Bewegen eines ersten Kolbens (6) von einer ersten Position in eine zweite Position innerhalb des ersten Zylinders (4) unter Verwendung einer Kolbenbetätigungseinrichtung, wobei sich das erste Fluid aus dem ersten Zylinder (4) über mindestens ein Fluidauslassventil (16) bewegt,
- Ansaugen des ersten Fluids aus dem Vorrat in den ersten Zylinder (4), indem sich der erste Kolben (6) von der zweiten Position zurück in die erste Position bewegt, wobei das erste Fluid über ein erstes Fluideinlassventil (14) einströmt und die Bewegung von der ersten Position in die zweite Position vorgespannt ist,
wobei ein Benutzer die Dosierung des Applikators (100) über ein erstes Dosiersteuerteil (20) einstellen kann, ; und
**gekennzeichnet durch** umfassen der weiteren Schritte,
- Einstellen vor dem Vorfüllen des ersten Zylinders (4) eine Dosierung des ersten Kolbens (6) innerhalb des ersten Zylinders (4) unter Verwendung des ersten Dosiersteuerteils (20), wobei das erste Dosiersteuerteil entlang einer Hauptachse des ersten Zylinders (4) in einer beliebigen einer Mehrzahl von Kerben, die jeweils einer Dosierungsmarkierung entsprechen, verschiebbar arretierbar ist, wobei das erste Dosiersteuerteil (20) an einem ersten Anschlagabschnitt (24) am ersten Kolben (6) anliegt, was wiederum eine erste Position des ersten Kolbens (6) und damit die Dosierung des ersten Fluids verändert, und
- fluidisches Verbinden einer Zufuhr eines zweiten Fluids mit dem Verteiler (54) des Applikators (100),
- Einstellen einer Dosierung eines zweiten Kolbens (7) innerhalb eines zweiten Zylinders (5) des Applikators (100) unter Verwendung eines zweiten Dosiersteuerteils (27), wobei das zweite Dosiersteuerteil (27) entlang einer Hauptachse des zweiten Zylinders (5) verschiebbar arretierbar ist, wobei das zweite Dosiersteuerteil an einem zweiten Anschlagabschnitt (36, 37) am zweiten Kolben (7) anliegt, was wiederum eine erste Position des zweiten Kolbens (7) und damit die Dosierung des zweiten Fluids verändert,
- Vorfüllen des zweiten Zylinders (5) unter Verwendung eines zweiten Hebelabschnitts (39),
- Auspressen eines zweiten Fluids aus dem zweiten Zylinder (5) durch Bewegen des zweiten Kolbens (7) von der ersten Position in eine zweite Position innerhalb des Zylinders unter Verwendung einer Kolbenbetätigungseinrichtung, wobei das zweite Fluid aus dem zweiten Zylinder über mindestens ein zweites Fluidauslassventil austritt,
- Ansaugen des zweiten Fluids aus der zweiten Zufuhr in den zweiten Zylinder (5) durch die Bewegung des zweiten Kolbens von der zweiten Position zurück in die erste Position, wobei das zweite Fluid über ein zweites erstes Fluideinlassventil einströmt und die Bewegung von der ersten Position in die zweite Position vorgespannt ist,
wobei ein Benutzer die Dosierung des Applikators (100) über das zweite Dosiersteuerteil (27) einstellen kann.

## Revendications

1. **Applicateur** (100) comportant :
- deux barillets ou plus comportant un premier barillet (4) ;
- un piston (6) mobile au sein du premier barillet (4) entre une première position variable et une deuxième position, dans lequel, en utilisation, un mouvement du piston (6) vers la première position peut aspirer un premier fluide dans le premier barillet (4), et un mouvement du piston (6) vers la deuxième position peut forcer le premier fluide à sortir du premier barillet (4) ;
- deux vannes d'entrée de fluide (14, 15) ou plus comportant une vanne d'entrée de fluide (14) pour permettre au fluide de s'écouler dans le premier barillet (4) au moins sous l'action du piston (6), ;
- deux vannes de sortie de fluide (16, 17) ou plus comportant une vanne de sortie de fluide (16) pour permettre au premier fluide de s'écouler en sortie du premier barillet (4) au moins sous l'action du piston (6) ;
- des moyens de sollicitation (18) pour solliciter le piston (6) vers la première position ;
**caractérisé en ce que** :
lesdits deux barillets ou plus comportent un deuxième barillet (5) ;
lesdites deux vannes d'entrée de fluide (14, 15) ou plus comportent une deuxième vanne d'entrée de fluide (15) pour permettre au deuxième fluide de s'écouler dans le deuxième barillet (5) ;
lesdites deux vannes de sortie de fluide (16, 17) ou plus comportent une deuxième vanne de sortie de fluide (17) pour permettre au deuxième fluide de s'écouler en sortie du deuxième barillet (5) ; dans lequel l'applicateur inclut en outre
- un deuxième piston (7) mobile entre une première position et une deuxième position au sein du deuxième barillet (5), dans lequel, en utilisation, un mouvement du deuxième piston (7) vers la première position peut aspirer un deuxième fluide dans le deuxième barillet (5), et un mouvement du deuxième piston (7) vers la deuxième position peut forcer le deuxième fluide à sortir du deuxième barillet (5) ;
- des deuxièmes moyens de sollicitation (19) pour solliciter le deuxième piston (7) vers la première position ;
- une partie de commande de dosage (20) qui coulisse de manière linéaire sur un axe longitudinal du premier barillet (4), et peut se verrouiller dans l'une quelconque d'une pluralité d'encoches (29) qui correspondent chacune à un repère de dosage pour buter contre une portion de butée (24) du piston qui à son tour définit la première position variable du piston (6) et par conséquent un dosage du premier fluide ;
- une deuxième partie de commande de dosage (21) qui coulisse le long d'un axe qui est parallèle à l'axe longitudinal du premier barillet, et peut se verrouiller dans l'une quelconque d'une pluralité d'encoches qui correspondent chacune à un repère de dosage pour buter contre une deuxième portion de butée (25) du deuxième piston qui à son tour définit la première position variable du deuxième piston et par conséquent un dosage du deuxième fluide ; et
- des moyens d'actionnement de piston servant à déplacer le piston (6) et le deuxième piston (7) vers leurs deuxièmes positions respectives pour distribuer le premier fluide et le deuxième fluide à partir de l'applicateur (100),
dans lequel un utilisateur peut régler le dosage de chacun du premier barillet et du deuxième barillet via la partie de commande de dosage (20) et la deuxième partie de commande de dosage (21), et administrer une dose du premier fluide et du deuxième fluide par les moyens d'actionnement de piston.

2. Applicateur (100) selon la revendication 1, dans lequel la portion de butée (24) du piston est sur le piston (6) ou reliée à celui-ci, et dans lequel la deuxième portion de butée (25) du deuxième piston est sur le deuxième piston (7) ou reliée à celui-ci.

3. Applicateur (100) selon l'une ou l'autre de la revendication 1 ou 2, dans lequel : (i) le piston (6) comporte une tête de piston (8) et une tige de piston (10) reliée à la tête de piston (8) ou solidaire de celle-ci, dans lequel la tige de piston (10) comporte la portion de butée (24) du piston (6) ; et (ii) le deuxième piston (7) comporte une deuxième tête de piston (9) et une deuxième tige de piston (11) reliée à la deuxième tête de piston (9) ou solidaire de celle-ci, dans lequel la deuxième tige de piston (11) comporte la portion de butée (25) du deuxième piston (7).

4. Applicateur selon l'une quelconque des revendications 1 à 3, dans lequel le piston (6) comporte une portion levier (38) qui est accessible depuis un extérieur de l'applicateur (100), et dans lequel le deuxième piston (7) comporte une deuxième portion levier (39) qui est accessible depuis un extérieur de l'applicateur (100).

5. Applicateur (100) selon la revendication 4, dans lequel la deuxième portion levier (39) permet à un utilisateur de déplacer le deuxième piston (7) indépendamment du premier piston (6).

6. Applicateur (100) selon la revendication 5, dans lequel la portion levier (38) et la deuxième portion levier (39) permettent à un utilisateur de déplacer chaque piston (6, 7) indépendamment des moyens d'actionnement de piston.

7. Applicateur (100) selon l'une quelconque des revendications 1 à 6, dans lequel le premier fluide pénètre dans le premier barillet (4) et le deuxième fluide pénètre dans le deuxième barillet (5) au niveau d'une première extrémité des deux barillets (4, 5) ou plus ou de manière adjacente à celle-ci, et le premier fluide sort du premier barillet (4) et le deuxième fluide sort du deuxième barillet (5) au niveau de la première extrémité ou de manière adjacente à celle-ci.

8. Applicateur (100) selon la revendication 7 dans lequel les deux vannes d'entrée de fluide (14, 15) ou plus sont adjacentes à une extrémité des deux barillets (4, 5) ou plus, et les deux vannes de sortie de fluide (16, 17) ou plus sont adjacentes à la même extrémité des deux barillets (4, 5) ou plus.

9. Applicateur (100) selon l'une ou l'autre de la revendication 7 ou de la revendication 8, dans lequel les deux vannes d'entrée de fluide (14, 15) ou plus et les deux vannes de sortie de fluide (16, 17) ou plus sont adjacentes à la première extrémité, et dans lequel l'applicateur comporte en outre une sortie (40), dans lequel la sortie (40) est à une deuxième extrémité des deux barillets (4, 5) ou plus, opposée à la première extrémité.

10. **Procédé** non thérapeutique **d'utilisation d'un applicateur** (100), comportant ou incluant les étapes de,
- raccordement fluidique de l'alimentation d'un premier fluide à un collecteur (54) de l'applicateur (100),
- amorçage d'un premier barillet (4) de l'applicateur à l'aide d'une première portion levier (38),
- forçage d'un premier fluide à partir du premier barillet (4) par le déplacement d'un premier piston (6) d'une première position à une deuxième position au sein du premier barillet (4), par l'utilisation de moyens d'actionnement de piston qui permettent au premier fluide de se déplacer à partir du premier barillet (4) via au moins une vanne de sortie de fluide (16),
- aspiration du premier fluide à partir de l'alimentation jusque dans le premier barillet (4) par le premier piston (6) revenant de la deuxième position vers la première position, le premier fluide se déplaçant vers l'intérieur via une première vanne d'entrée de fluide (14), le mouvement de la première position vers la deuxième position étant sollicité,
dans lequel un utilisateur peut régler le dosage de l'applicateur (100) via une première partie de commande de dosage (20) ; et
**caractérisé par le fait qu'**il comporte en outre les étapes de,
- avant l'amorçage du premier barillet (4), ajustement d'un dosage du premier piston (6) au sein du premier barillet (4) en utilisant la première partie de commande de dosage (20), où la première partie de commande de dosage est apte à être verrouillée par coulissement le long d'un axe principal du premier barillet (4) dans l'une quelconque d'une pluralité d'encoches qui correspondent chacune à un repère de dosage, la première partie de commande de dosage (20) étant en butée contre une première portion de butée (24) sur le premier piston (6) qui à son tour fait varier une première position du premier piston (6) et par conséquent le dosage du premier fluide, et
- raccordement fluidique d'une alimentation d'un deuxième fluide au collecteur (54) de l'applicateur (100),
- ajustement d'un dosage d'un deuxième piston (7) au sein d'un deuxième barillet (5) de l'applicateur (100) à l'aide d'une deuxième partie de commande de dosage (27), où la deuxième partie de commande de dosage (27) est apte à être verrouillée par coulissement le long d'un axe principal du deuxième barillet (5), la deuxième partie de commande de dosage étant en butée contre une deuxième portion de butée (36, 37) sur le deuxième piston (7) qui à son tour fait varier une première position du deuxième piston (7) et par conséquent le dosage du deuxième fluide,
- amorçage du deuxième barillet (5) à l'aide d'une deuxième portion levier (39),
- forçage d'un deuxième fluide à partir du deuxième barillet (5) par le déplacement du deuxième piston (7) de la première position à une deuxième position au sein du barillet, par l'utilisation de moyens d'actionnement de piston qui permettent au deuxième fluide de se déplacer à partir du deuxième barillet via au moins une deuxième vanne de sortie de fluide,
- aspiration du deuxième fluide à partir de la deuxième alimentation jusque dans le deuxième barillet (5) par le deuxième piston revenant de la deuxième position vers la première position, le deuxième fluide se déplaçant vers l'intérieur via une deuxième vanne d'entrée de fluide, le mouvement de la première position vers la deuxième position étant sollicité,
dans lequel un utilisateur peut régler le dosage de l'applicateur (100) via la deuxième partie de commande de dosage (27).
